# EUROPÄISCHE PATENTANMELDUNG

(11) **EP 2 702 984 A1**
(43) Veröffentlichungstag der Anmeldung: **05.03.2014**
(21) Anmeldenummer: 12401180.0
(22) Anmeldetag: 27.08.2012
(51) Int. Cl.: A61K 9/70, A61K 31/165, A61L 15/44

(54) **Wirkstoffhaltiges Pflaster zur Therapie lokaler Symptome**

(71) Anmelder: MSF Medizinische Sportwerke Frankfurt GmbH, 60487 Frankfurt (DE)
(72) Erfinder: Hofmann, Wolfgang, 60487 Frankfurt (DE)
(74) Vertreter: Zdarsky, Stefan

(57) **Zusammenfassung**

Die Erfindung betrifft mit wirkstoffhaltiger Klebmasse beschichtete Pflaster, die zur lokalen Therapie von Schmerzen (zumeist Schmerzen bedingt durch Verspannungen aller Art, Rheuma, Prellungen, Zerrungen oder Arthrose, Kopfschmerzen, Migräne) eingesetzt werden. Darüber hinaus betrifft die Erfindung eine neuartige lokale Therapie für Symptome, insb. im Rahmen der Schmerztherapie.

Es werden neuartige, bisher nicht in Wirkstoffpflastern, insbesondere Wärme-, Kälte- und Schmerzpflastern verwendete Dimensionen und geometrische Formen beschrieben, die dazu geeignet sind, die Schmerzzone nicht vollständig bzw. homogen abzudecken, sondern durch einzelne oder mehrere schmale Streifen oder andere geometrische Formen in regelmäßigen und unregelmäßigen Abständen zu unterbrechen bzw. punktuell/segmental zu stimulieren und so eine regional und lokal modulierte Schmerztherapie zu ermöglichen.

## Beschreibung

Die Erfindung betrifft ein Pflaster mit Wirkstoffbeschichtung (wirkstoffhaltige Klebmasse), bestehend aus einer der folgenden Substanzgruppen, oder aber bestehend aus einer Kombination von
a. lokal die Durchblutung fördernde, das Gefühl von Wärme vermittelnde, Arthrose lindernde, krampflösende oder/und antirheumatische Substanzen, insb. Capsaicin, andere Capsaicioide oder Nonivamid, die u.a. am TRPV1 Kanal wirken, oder Durchblutung fördernde, Wärme vermittelnde ätherische Öle und pflanzliche Extrakte, insb. Ackerminze, Angelika, Angelikawurzel, Anis, Arnika, Baldrian, Basilikum, Bay, Beifuss, Benzoe, Bergamotte, Bohnenkraut, Cajeput, Cananga, Cassia, Davana, Douglasie, Edeltanne, Estragon, Eukalyptus, Fenchel, Fichte, Fichtennadel, Galbanum, Geranie, Gingergrass, Guajakholz, Ingwer, Johanniskraut, Kamille, Kampfer, Kardamom, Kiefer, Kiefernadelöl, Koriander, Kümmel, Latschenkiefer, Lavendel, Lemongrass, Liebstöckel, Limette, Lorbeer, Mairose, Majoran, Mandarine, Manuka, Meerkiefer, Melisse, Minze, Muskatellersalbei, Muskatnuss, Myrte, Nelke, Niaouli, Orange, Oregano, Origanum, Pampelmuse, Pfeffer, Pfefferminze, Rainfarn, Rosenholz, Rosmarin, Römische Kamille, Salbei, Sandelholz, Sassafras, Schafgarbe, Schopflavendel, Sellerie, Tabak, Teebaum, Terpentin, Thuja, Thymian, Wacholder, Wacholderbeere, Weisstanne, Wermut, Wiesenkönigin, Wintergreen, Ysop, Zeder, Zirbelkiefer, Zitrone, Zitronenmelisse, Zypresse
b. lokal das Gefühl von Kälte vermittelnde oder kühlende Substanzen, insb. Menthol, Minzöle, Campher, Methyl Salicylat (Wintergrünöl), andere ätherische Öle und pflanzliche Extrakte, die u.a. am Kälte-Menthol Rezeptor (TRPM8) wirken oder auf sonstige Weise das Gefühl von Kälte vermitteln
c. lokal wirkende, nicht opioide oder auch opioide Analgetika, z.B. Diclofenac oder Lidocain, andere transkutan verwendbare Analgetika

Die Erfindung befasst sich mit dem Problem, dass derzeit für die lokale Schmerzbehandlung vermittels Pflaster (Wärmepflaster, kühlende Pflaster oder Analgetika-Pflaster, jeweils mit wirkstoffhaltiger Klebstoff-Beschichtung) nur großflächige und/oder bis auf kleinere (ca. 3 mm große) Belüftungslöcher durchgehend abdeckende Behandlungen in Form rechteckiger, annähernd quadratischer wirkstoffhaltiger Pflaster bekannt sind (Verhältnis der längeren zur kürzeren Seite zwischen 1:1 und maximal 2:1, wobei das Seitenverhältnis 2:1 in der Praxis nie erreicht und angewandt wird; marktüblich sind Seitenverhältnisse von 1,4 bis 1,6 zu 1), jedoch im Unterschied zur gängigen Überzeugung, nur eine zentral flächige Überklebung des Schmerzareales sei therapeutisch nützlich, ein Bedarf für kleinflächigere, insbesondere länglich geformte streifenförmige, und multi-lokal wirkstoffhaltige Pflasterbehandlungen besteht, die gerade nicht das Schmerzareal homogen flächig mit einem monotonen Stimulus überlagern, sondern das Schmerzareal auf verschiedene Weise überschneiden oder punktuell darin liegen.

Die marktüblichen, größer flächigen bzw. in einer homogenen Fläche über der Schmerzregion eingesetzten Pflaster mit einem Seitenverhältnis von 1:1 bis deutlich unter 2:1, typischerweise 1,4 bis 1,6:1, sind in ihrer Form und Größe in mehrfacher Hinsicht nachteilig.

Zunächst wird durch die große Fläche des Pflasters eine ebenso große Hautfläche mit dem Pflaster abgedeckt und dem abgegebenen Wirkstoff ausgesetzt. Dies führt besonders im Falle der Wärmepflaster zu einer erheblichen Reizung des betreffenden Areals, das sich in lokalen oberflächlichen Schmerzen, einem subjektiven Gefühl der übermäßigen Hitze, sowie oft großflächigen Rötungen und Schwellungen niederschlägt. Eine "vollflächige Verklebung" führt zudem zu stärkerer Okklusion der Haut. Der normale Wasserabgabeprozess wird großflächig gestört. Dies kann negative Folgen für den betroffenen Hautabschnitt haben, insbesondere wenn über lange Zeit immer dieselbe Fläche verschlossen wird. Die subjektive Wärme- und Hitzewahrnehmung ist zwar Teil der gewünschten Durchblutungsförderung, ausgelöst durch die durchblutungsfördernden Substanzen. Die Größe und Form der handelsüblichen Pflaster jedoch sind in ihrer eher groben, annähernd quadratischen Form und Ausführung nur dazu geeignet, dem lokalen, durch Verspannung, Rheuma oder andere Faktoren bedingten Schmerz mit einem monotonen, auf der ganzen zu behandelnden Fläche gleichen, undifferenzierten therapeutischen Stimulus zu begegnen. Hierbei wird die insbesondere bei Wärmepflastern entstehende Wärme durch die Großflächigkeit häufig als unangenehm empfunden.

Aufgrund der oben beschriebenen Hautreizungen sollte gerade bei Wärmepflastern die Haut nicht über längere Zeit wiederholt dem Wirkstoff ausgesetzt werden. Dies ist bei einer homogen abdeckenden Therapie nur durch regelmäßige Therapieunterbrechungen möglich, die letztlich den Therapieerfolg reduzieren.

Als Wichtigstes erscheint in überraschender Analogie zu neueren Erkenntnissen über traditionelle Heilmethoden und wie in klinischen Versuchen gezeigt (siehe nachfolgend), gerade die homogen gleichförmige, gegenüber einer lokal begrenzten, innerhalb des Schmerzbereiches unterschiedliche Zonen differenziert ansprechenden, und damit modulierbaren Therapie, nachteilig.

Mehrere etablierte Techniken der Chinesischen Medizin beschäftigen sich seit Jahrtausenden mit Methoden, Schmerzen und andere in der Tiefe des Körpers liegende medizinische Zustände durch gezielte lokale Reizungen der Körperoberfläche zu therapieren. Dies geschieht zum Beispiel in der Akupunktur (Reizung mit Hilfe von Nadeln), der Akupressur (Reizung mit stumpfem Druck), sowie der Moxibustion (lokale, selektive Reizung von Hautarealen mit Wärme). Neuerdings wird auch mit Infrarot-Lasern lokal gereizt (Laser-Akupunktur).

Klinisch erwiesen ist in großen Studien (z.B. die German Acupuncture Trials 2002-2007, GERAC) die Wirksamkeit dieser Techniken bei Kniegelenksarthrose und dadurch bedingten Schmerzen, bei chronischen tiefen Rückenschmerzen und bei der Prophylaxe von Migräne-Attacken. In der Tat ist in Studien die angewandte lokale Reiztherapie häufig der klassischen, schulmedizinischen, medikamentösen Schmerzbehandlung deutlich überlegen. Aufgrund dieser Ergebnisse ist seit 2007 die Akupunktur bei Rückenschmerzen und chronischen Gelenkschmerzen in Deutschland Teil der Kassenleistung. Auch die international anerkannten "Cochrane Reviews" kommen zu dem Schluss, dass die "Akupunktur bei Migräne mindestens so wirksam, möglicherweise auch wirksamer als medikamentöse prophylaktische Therapie ist, und dies bei geringeren unerwünschten Wirkungen".

Die meisten traditionellen Anleitungen gehen davon aus, dass spezielle, genau definierte Orte der Wirkung existieren, die gereizt werden müssen, um eine besondere neurologisch-sensorische Wirkung zu entfalten. So existieren weit verbreitete Karten, die hoch spezifische Wirkorte bzw. Reizpunkte darstellen. Gerade diese Spezifität wird aber in den neueren einschlägigen Studien bezweifelt bzw. widerlegt. So zeigt sich in diesen Studien, dass die zu reizenden Areale keineswegs so spezifisch sind, dass eine Wirkung ausschließlich einem genau anatomisch definierten, kleinen Areal zuzuschreiben ist. Diejenige Akupunktur, bei der an irgendwelchen Orten in der Nähe des zu therapierenden Areals gestochen oder z.B. auf kleinen Arealen mit Wärme gereizt wird, ist genauso effektiv wie die "echte" Akupunktur, Akupressur oder Moxibustion. Neurologisch wirksamer zu sein scheint aber durchweg die Reizung an verschiedenen kleinen Arealen auf dem Schmerzfokus und um den Schmerzfokus herum, an Stelle einer unselektiven, großflächigen Reizung.

Häufig werden die zitierten Wirkungen der Chinesischen Medizin der Placebowirkung zugeschrieben; jedoch ist inzwischen anerkannt, dass es sich hier um echte Wirkungen handelt, die aber weniger spezifisch sind als früher angenommen. Nach heutigem Verständnis gehören diese Bereiche zu den "Umsteuerungs-" und "Regulationstherapien".

Während die Spezifität des Wirkortes kleiner ist als früher angenommen, ist doch gesichert, dass eine allzu großflächige Reizung mit vielen Nadeln, Druck- oder Wärmepunkten mit der entstehenden lokal undifferenzierten sensorischen Reizung weniger wirksam ist.

Die hier vorliegende Erfindung ermöglicht überraschenderweise eine neuartige Form von Therapien mit wirkstoffhaltigen Pflastern, insbesondere für Pflaster mit wärmender, kühlender oder lokal direkt schmerzstillender Wirkung. Ein Erklärungsmodell für die beschriebenen überraschenden Wirkungen ist die Parallele zu den oben beschriebenen Regulationstherapien.

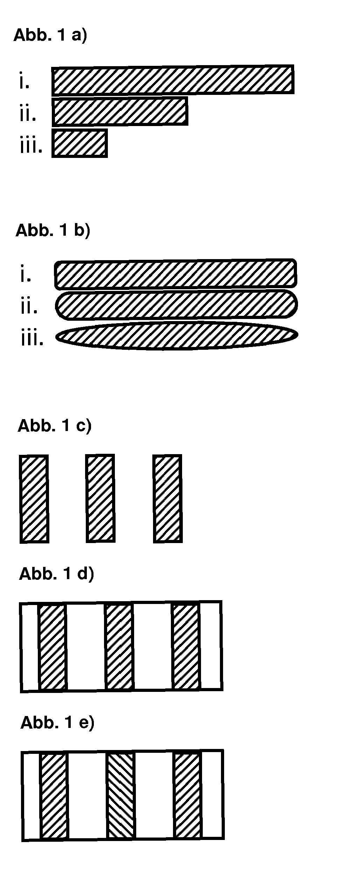

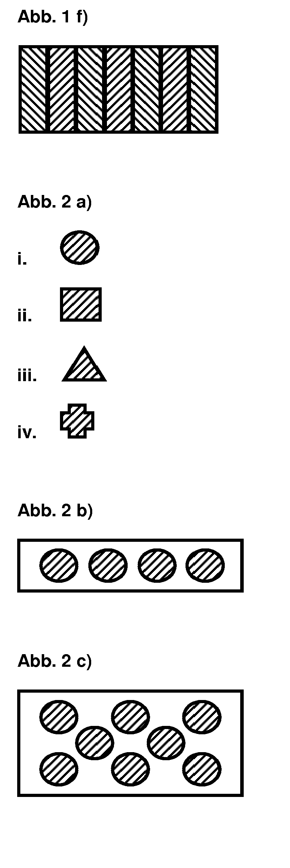

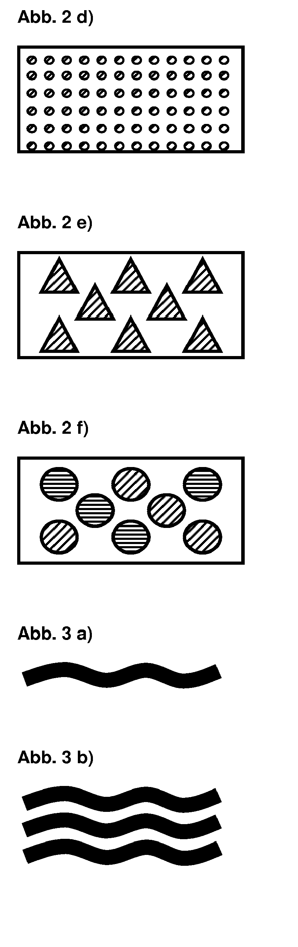

Im Unterschied zu den flächig homogen abdeckenden Wirkstoffpflastern, die in der Schmerzregion nicht moduliert in die Wahrnehmung des Schmerzes eingreifen und dadurch mit viel Einsatz des Wirkstoffes bestenfalls eine dumpfe Breitenwirkung entfalten, sind Pflaster nach dieser Erfindung vorzugsweise bandförmig. Die Breite liegt bevorzugt bei 0,5 bis 6 cm Länge des Bandes, wobei die längere Seite vorzugsweise mindestens 2 mal so lang ist wie die kürzere (s. Abb. 1a/1b, jeweils i-iii). Wirksam sind auch beliebige Geometrien, insb. Punkte, Kreise, Sterne etc. (s. Abb. 2a), wobei die maximal abgedeckte Seitenbreite eines geometrischen Elementes vorzugsweise nicht mehr als 6 cm betragen sollte (Geometriebeispiele siehe Abbildungen 2-5). Dies erlaubt durch ihre Ausgestaltung und Anwendung eine differenzierte neurologisch-sensorische Modulation des Schmerzes, bei der der zu behandelnde Bereich durch kleinere, räumlich verteilte Einzel-Stimuli in Form von Pflaster-Segmenten unterteilt wird, ähnlich den beschriebenen Methoden der Chinesischen Medizin (Akupunktur, Akupressur, Moxibustion). Hierdurch wird die gleiche oder sogar eine bessere Wirkung erzielt als mit der großflächigen Therapie.

Weiterhin kann der Schmerz mit Hilfe der beschriebenen Anordnungen überraschenderweise ohne Unterbrechung und Behandlungspause therapiert werden: Nach einem Tag Therapie z.B. auf einem Hautareal mit drei parallelen Wirkstoffpflasterstreifen mit einem jeweiligen Abstand von einer Streifenbreite (Abb. 1c) kann am folgenden Tag die Therapie mit Wirkstoffpflasterstreifen in den vorher jeweils frei gebliebenen Arealen fortgesetzt werden. Dies wird darauf folgend fortlaufend abgewechselt. Es kann auch eine Therapie mit einem einzelnen Streifen durchgeführt werden, wobei bei der zweiten Anwendung typischerweise eine ebenfalls in der Nähe der Schmerzquelle liegende Parallele zum ersten Streifen gewählt wird. Hierdurch wird insgesamt bei maximalem Therapieerfolg durch die segmentale Therapie des Schmerzareales eine Reduktion unerwünschter Nebenwirkungen wie übermäßige Hitze, andere Überreizungen, Rötungen, Schwellungen etc. erreicht. Auch wird gegenüber herkömmlichen Behandlungsmethoden nicht immer dieselbe Hautstelle gereizt. Die jeweils behandelte Hautpartie kann sich erholen. Längerfristige Behandlungen werden so ermöglicht, ohne die Therapieerfolge als solche zu gefährden, sondern im Gegenteil: Sie werden verbessert oder überhaupt erst ermöglicht.

Optisch können die beschriebenen bandförmigen Ausgestaltungen Ähnlichkeit mit den in der Kinesiologie (einem in den 1960er Jahren in den USA entwickelten Ansatz zur Therapie von Verspannungen) verwendeten Bändern aufweisen. Sie unterscheiden sich aber darin, dass bei den Bändern in der Kinesiologie typischerweise kein Wärme oder Kälte vermittelnder oder lokal schmerzstillender Wirkstoff eingesetzt wird.

Beschrieben sind mit der hier vorliegenden Erfindung Anwendungen von Wärme-oder Kältereiz auslösenden oder die Durchblutung fördernden Wirksubstanzen bzw. pflanzliche Extrakte, oder aber direkt schmerzstillenden Medikamenten (siehe Anspruch zu 1) in Pflasterapplikationen mit wirkstoffhaltigen Klebmassen, die in ihrer Form dazu geeignet sind, Schmerzbereiche in Segmente zu unterteilen und so auf kleinere Zonen innerhalb des größeren Schmerzareals therapeutisch einzuwirken. Dies macht sich in einem Erklärungsmodell die Wirkweise der traditionellen Chinesischen Medizin zunutze, indem es auf kartographisch genaue Anwendung verzichtet, dem Anwender aber Module zur individuellen, lokalen bzw. multi-lokalen und gerade nicht gleichmäßigen, homogen flächigen Schmerzüberlagerung an die Hand gibt.

Denkbar sind in diesem Zusammenhang nicht nur wirkstoffhaltige Pflaster mit wärmenden Substanzen, wie Capsaicinoiden, sondern auch kühlende Effekte, wie sie zum Beispiel durch Menthol hervorgerufen werden oder aber direkt schmerzstillende Effekte, wie z.B. die von Diclofenac.

Hierbei bewirkt die Anwendung von Wärme-stimulierenden Reizen eine Zunahme des allgemeinen Entspannungsgefühls, eine Senkung des Muskeltonus, eine Zunahme der Durchblutung durch Gefäßweitstellung und eine Analgesie durch die oben beschriebenen Mechanismen. Die Anwendung von Kälte-stimulierenden bzw, das Gefühl von Kälte vermittelnden Reizen dagegen bewirkt eine Erhöhung der Wachsamkeit und des Muskeltonus, eine Abnahme der Durchblutung durch Engstellung der Gefäße, gefolgt von einer reaktiven Hyperämie (Zunahme der Durchblutung nach Kältereiz), einer Kälteanalgesie sowie einer Entzündungshemmung. Die direkte Anwendung bestimmter opioider oder nicht opioider Analgetika wirkt schmerzstillend und entzündungshemmend. Diese Substanzen können allein oder in Kombination (wie in den Ansprüchen beschrieben) zur lokal modulierenden Umsteuerungs- und Regulationstherapie, wie oben und in den Ansprüchen beschrieben, verwendet werden.

Eine Kombination von Wirkstoffen derart, dass sie nicht gemeinsam in der gleichen Wirkstoffschicht vorliegen, sondern nebeneinander in punktuellen Anordnungen oder in parallelen, sich kreuzenden Streifen, kann sinnvoll sein, um durch die sich teilweise sensorisch widersprechenden Reize eine umso stärkere neuronale Antwort und therapeutische Wirkung zu erzielen (s. Abb. 1f, 2f, 4a-c).

Die oben beschriebenen Effekte konnten in Versuchen an klinischen Probanden bestätigt werden (Näheres hierzu unter "Vorteile der Erfindung").

### Vorteile der Erfindung

1. Die hier vorliegende Erfindung macht sich die Erkenntnis zunutze, dass in Versuchen festgestellt werden konnte, dass eine Behandlung von Schmerzen, verursacht insb. durch Verspannungen aller Art, Rheuma, Prellungen, Zerrungen, Arthrose oder Migräne (auch zur Vorbeugung der letzteren), mit einzelnen oder mehreren kleinflächigen bzw. streifenförmigen oder geometrisch angeordneten Abschnitten von Pflastern mit wirkstoffhaltiger Klebmasse (nachfolgend: Wirkstoffpflaster), die jeweils mit einem geeigneten Wirkstoff versehen sind, eine sehr erfolgreiche Schmerztherapie ermöglicht. Diese überraschende Beobachtung erklärt sich in einem möglichen Modell aus der dargestellten Analogie zur Wirkweise der traditionellen Chinesischen Medizin.
Bedeckt man eine Schmerzzone nicht mit einem großflächigen oder (bis auf kleine Belüftungslöcher) weitgehend homogen das Schmerzareal überlagernden Wirkstoffpflaster, sondern therapiert sie zonal mit einzelnen oder mehreren schmaleren Streifen bzw. anderen Anordnungen des Pflasters (s. Abb. 1-5), so konnte in Versuchen eine erfolgreiche Behandlung des Schmerzreizes erzielt werden. Eine erfolgreiche Schmerztherapie ist somit nicht nur durch eine Behandlung der Schmerzen durch einen monoton gleichförmigen neurologischen Stimulus des Zentrums und der Umgebung der Schmerzquelle möglich. Vielmehr scheint es auch möglich und sogar effektiver zu sein, die Schmerzausbreitung durch die neurologisch-sensorische Einwirkung auf kleinere, lose im größeren Schmerzareal liegende Flächen zu unterbinden. Diese Behandlungsweise führt offenbar zu einer effektiven Unterbrechung der Schmerzwahrnehmung und des Schmerztransportes. Hierdurch kommt es in der Folge nicht nur zu einer Schmerzreduktion unmittelbar an der therapierten Stelle, sondern auch zu einer Entspannungswirkung und Schmerzreduktion in den entfernteren Partien. So führt diese Behandlungsform zu einer Entspannung der gereizten Körperpartien und letztlich zu einer effektiven Behandlung des zentralen Schmerzpunktes/-areals selbst.
Es hat sich herausgestellt, dass Verspannungen und Schmerzen mit der im Folgenden beschriebenen Behandlungsmethode erfolgreich behandelt werden konnten:
Man bringt einen (s. Abb. 1a/b) oder mehrere schmale Streifen des Wirkstoffpflasters (bei mehreren Streifen sind diese idealerweise parallel zu einander mit einem Abstand einer Pflasterbreite, s. Abb. 1c), quer zur Hauptschmerzrichtung auf die schmerzenden Bereiche auf. Die Behandlung mit solchen schmalen Streifen scheint zu bewirken, dass der Schmerzreiz unterbrochen und der Schmerztransport gestoppt wird. Es kommt zu einer deutlichen Schmerzreduktion und Entspannung in diesen Bereichen. Diese Schmerzlinderung und Entspannungswirkung setzt sich in die nicht behandelten Bereiche fort. So wird eine schnelle Linderung bis hin zur vollständigen Beseitigung des Schmerzes bewirkt.
2. In ausführlichen Versuchen wurden kommerziell erhältliche, mit wirkstoffhaltiger Klebmasse beschichtete Pflaster (Pflaster mit einem oder mehreren der beschriebenen Wirkstoffe) herkömmlicher Dimensionen, zu geeigneten Dimensionen und geometrischen Formen, wie hier beschrieben, zurechtgeschnitten und daraufhin an Patienten mit Schmerzen, zumeist verursacht durch Verspannungen, angewendet.
Patienten und der anwendende Arzt beurteilten mit Hilfe von VAS Skalen verschiedene klinische Parameter. VAS Angaben wurden unmittelbar vor der Anwendung erhoben (Tag Null) sowie 1-12 Stunden in 2 Stunden-Intervallen und an 7 aufeinander folgenden Tagen nach dem Beginn des Versuchs bzw. bis zum Abbruch oder der Beendigung der Therapie nach Erreichen des maximalen Therapieerfolges. Wann immer möglich wurden symmetrisch vergleichende Beobachtungen durchgeführt, d.h. auf der einen Seite die klassische breitflächige Anwendung (nachfolgend: "Anwendung A"), auf der anderen Seite eine Anwendung nach den hier beschriebenen Ansprüchen (schmale, längliche Streifen oder multiple Punkte) (nachfolgend: "Anwendung N").
Pflaster wurden jeweils für 6-12 Stunden auf die Anwendungsbereiche aufgeklebt und danach entfernt. Im Falle der klassischen Anwendung erfolgte die Auflage des neuen Pflasters auf der gleichen Hautstelle entsprechend der in der Patienten- bzw. Ärzte-Information vorgeschriebenen Methode nach einer Erholungsphase von 12 Stunden, in der keine Pflaster-Anwendung stattfand. Im Falle der hier beschriebenen neuartigen Anwendung erfolgte die Auflage der jeweils neuen Pflaster ohne Unterbrechung jeweils in den Lücken zwischen den in der vorherigen Anwendung beklebten Hautstellen, bzw. bei Verwendung einzelner Streifen der nachfolgende jeweils parallel zum vorherigen.
Verglichen mit der klassischen Anwendung (Anwendung A), fällt zunächst die wesentlich größere Patientenfreundlichkeit der beschriebenen neuen Anwendung N auf, gemessen an vorzeitigen Abbrüchen und Brennen nach VAS Skalen Angaben. Vorzeitige Behandlungsabbrüche (vor Ablauf der 12 Stunden Behandlungsdauer auf der jeweils gleichen Hautstelle) waren über 10 Mal so häufig bei Anwendung A, verglichen mit Anwendung N. Ebenso verweigerten Probanden deutlich häufiger die Anwendung des folgenden Pflasters bei Anwendung A, verglichen mit Anwendung N. In letzterer kam der vollständige Behandlungsabbruch gar nicht vor. Rötungen der Haut nach Abnahme der Pflaster sind in beiden Methoden vergleichbar, aber offenbar ist durch die kleinere, jeweils an einem Stück beklebte Fläche das daraus entstehende Brennen bei Therapie N geringer als in Therapiemethode A, was zu einer besseren Verträglichkeit und damit Akzeptanz der Behandlungsmethode auch über längere Zeit führt.
Wirkungen, gemessen an Reduktion des Schmerzes, der Verspannung und/oder Funktionseinschränkungen sind nach der VAS Skala überraschenderweise ebenfalls signifikant besser bei Anwendung N, verglichen mit Anwendung A. Hier zeigte sich sowohl ein besseres Gesamtergebnis als auch wesentlich schnelleres Erreichen dieses Ergebnisses. Letzteres ist nach ärztlicher Einschätzung zurückzuführen auf die Möglichkeit der zeitlich unterbrechungsfreien Behandlung, in Kombination mit den diskutierten neuartig beschriebenen Mechanismen der lokalisierten, zonal modulierenden Therapie.
Insgesamt ist die Zufriedenheit sowohl unter Patienten als auch bei ärztlichen Beobachtern bei Behandlung mit der neuen Therapie (Anwendung N) deutlich grösser als unter der klassischen Therapie (Anwendung A).
Das angewandte Schema stellt sich beispielhaft wie folgt dar (Tab. 1): Verwendet wurde stets eine VAS Skala von 1-100). Jeweils Beobachtung an Tag 0 sowie in verschiedenen Intervallen nach Beginn der Behandlung wie im Text beschrieben.

**Tab. 1: Bewertungsschema für Pflaster Anwendungen**

| **Anwendung A** | **Anwendung N** |
|---|---|
| | |

| **Patient subjektiv:** | **Patient subjektiv:** |
|---|---|
| Schmerz | Schmerz |
| Verspannung | Verspannung |
| Funktionseinschränkung | Funktionseinschränkung |
| Brennen | Brennen |
| Zufriedenheit insgesamt | Zufriedenheit insgesamt |
| | |

| **Arztliche Einschätzung:** | **Arztliche Einschätzung:** |
|---|---|
| Rötung | Rötung |
| Lokale Verspannung | Lokale Verspannung |
| Funktionseinschränkung | Funktionseinschränkung |
| Zufriedenheit insgesamt | Zufriedenheit insgesamt |

Die Ergebnisse lassen sich tabellarisch wie folgt zusammenfassen (Tab. 2):

**Tab. 2: Zusammengefasste Ergebnisse der klinischen Tests**

| | | Anwendg. A (traditionell) | Anwendg.N (neu) |
|---|---|---|---|
| Nebenwirkungen | | | |
| | Abbrüche vor 12 Stunden (in %) | 35 | 3 |
| | Durchschnittl. Behandlungsdauer der Einzelanwendung (in Stunden) | 4 | 11,5 |
| | Ablehnung der Weiterbehandlung nach erster Anwendung (in %) | 5 | 0 |
| | Brennen 3 Stunden nach neuer Pflaster Applikation (VAS 1-100) | 56 | 27 |
| | Rötung 3 Stunden nach neuer Pflaster Applikation (VAS 1-100) | 31 | 30 |
| | | | |

| Wirkungen | | | |
|---|---|---|---|
| | Zu therapierender Schmerz 3 Tage nach erster Pflaster Applikation (VAS Reduktion 1-100) | 29 | 38 |
| | Zeit bis 50% der Maximalwirkung (in Tagen) | 4 | 3 |
| | Funktionseinschränkung (nur bei Verspannung) 3 Tage nach erster Pflaster Applikation (VAS Reduktion 1-100) | 26 | 41 |
| | Zeit bis 50% der Maximalwirkung (in Tagen) | 4 | 2 |
| | Zufriedenheit insgesamt 7 Tage nach erster Behandlung (VAS 1-100) | 41 | 72 |

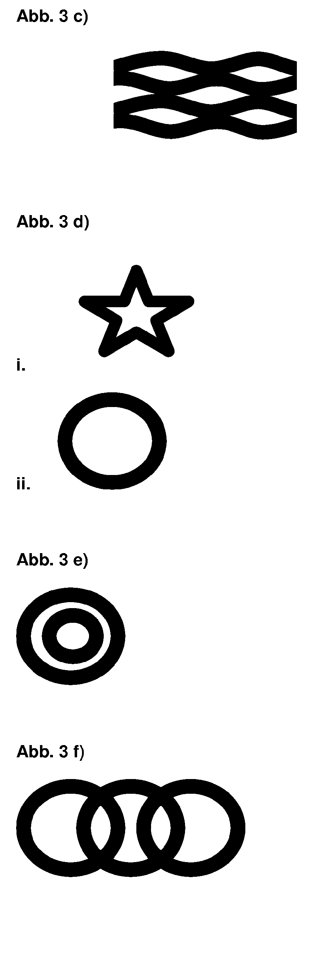

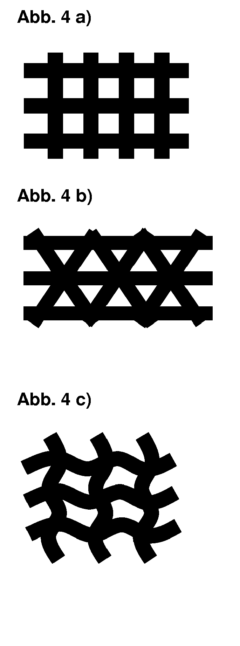

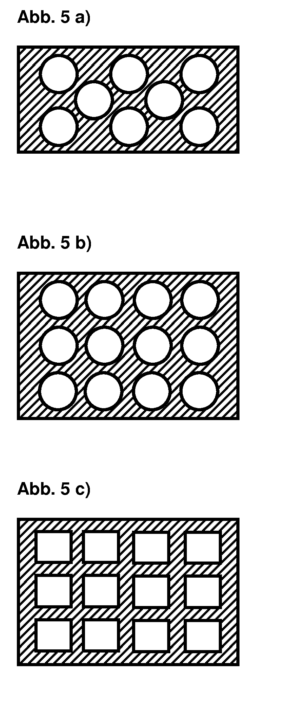

3. Es hat sich gezeigt, dass die Pflaster bei Streifen in der Einzelanwendung bevorzugt eine Breite von nicht weniger als 0,5 cm aufweisen sollten, bei der Anwendung in punktueller Form nicht weniger als 0,2 cm. Die bevorzugte Breite der Pflasterstreifen oder Punktgröße ist abhängig von Größe und Intensität der Schmerzquelle. Eine Breite von mehr als 6 cm ist jedoch regelmäßig nicht förderlich für die Erzielung des hier beschriebenen Effektes. Der Abstand zwischen den Pflasterstreifen ist abhängig von der Größe der zu behandelnden Schmerzquelle und sollte etwa die Breite eines Pflasterstreifens betragen. Der Abstand sollte jedoch 6 cm nicht übersteigen.
Unter den streifenförmigen Geometrien hat sich ein Seitenverhältnis der wirkstoffhaltigen Klebmasse von 0,5 bis 6 cm Breite zu 1 bis 100 cm Länge als vorteilhaft erwiesen, wobei das Verhältnis der längeren zur kürzeren Seite mindestens einem Verhältnis von 2:1 entsprechen sollte (längere Seite mindestens 2 mal so lang wie die kürzere). Besonders vorteilhaft waren Dimensionen von 4 bis 6 cm Breite und 8 bis 100 cm Länge, ganz besonders vorteilhaft waren Dimensionen von 4,5 bis 5,5 cm Breite und 9 bis 100 cm Länge.
Sehr vorteilhaft waren ebenso Dimensionen von 0,5 bis 2,5 cm Breite und 1 bis 100 cm Länge, wobei das Verhältnis der längeren zur kürzeren Seite mindestens einem Verhältnis von 2:1 entsprechen sollte. Hierin besonders vorteilhaft waren Dimensionen von 1 bis 2 cm Breite und 2 bis 100 cm Länge.
Ausgesprochen vorteilhaft waren auch Dimensionen von 2,5 bis 4,5 cm Breite und 5 bis 100 cm Länge, wobei das Verhältnis der längeren zur kürzeren Seite mindestens einem Verhältnis von 2:1 entsprechen sollte. Unter diesen besonders vorteilhaft waren Dimensionen von 3 bis 4 cm Breite und 6 bis 100 cm Länge.
In Versuchen hat sich gezeigt, dass bei Verwendung mehrerer Streifen eine parallele Ausrichtung der Streifen mit jeweils einer Streifenbreite Abstand zwischen den Streifen die besten Behandlungsergebnisse erzielt (s. Abb. 1c). Dies ist aber nicht zwingend erforderlich. Vielmehr sollte die Anordnung in einer Weise erfolgen, dass die Pflaster möglichst orthogonal zur Richtung der Schmerzausbreitung angebracht werden.
Bei strahlenförmig sich ausbreitenden Schmerzen hat es sich als vorteilhaft erwiesen, die jeweiligen Hauptstrahlen mit Pflastern in der hier beschriebenen Weise orthogonal zu unterbrechen.
Der Schmerz kann mit Hilfe der beschriebenen Anordnungen ohne Unterbrechung und Behandlungspause therapiert werden: Nach einem Tag Therapie z.B. auf einem Hautareal mit drei parallelen Wirkstoffpflasterstreifen mit einem jeweiligen Abstand von einer Streifenbreite (s. Abb. 1c) kann am folgenden Tag die Therapie mit Wirkstoffpflasterstreifen in den vorher jeweils frei gebliebenen Arealen fortgesetzt werden. Dies wird darauf folgend fortlaufend abgewechselt. Hierdurch wird insgesamt bei maximalem Therapieerfolg durch die Segmentale Therapie des Schmerzareales eine Reduktion unerwünschter Nebenwirkungen, wie übermäßige Hitze, andere Überreizungen, Rötungen, Schwellungen etc., erreicht.
Es hat sich gezeigt, dass es gerade bei sehr unspezifisch streuenden Schmerzen vorteilhaft ist, die Bereiche wirkstoffhaltiger Klebmasse in sich orthogonal kreuzenden Streifen, also in Form eines Gitter- oder Punktenetzes, aufzubringen. Dieses Gitter- oder Punktenetz kann je nach Intensität des Schmerzes in beliebiger Größe zugeschnitten werden (Abb. 4a-c / 5a-c); denkbar sind auch nicht oder nur an wenigen Stellen kreuzende Geometrien (s. Abb. 3d-f). Es empfiehlt sich aus anwendungspraktischen Gesichtspunkten, die jeweilige Streifenbreite der Wirkstoffstreifen auf 0,3 bis 3,0 cm zu begrenzen.
Als neurologisch-sensorischer Stimulus sehr geeignet sind auch viele andere geometrische Formationen des Wirkstoffpflasters (Punkte, Kreise, Sterne, Dreiecke etc., beispielhaft Abb. 2 und 3) sowie größere, aus vielen kleinen Unterstrukturen zusammengesetzte Formen (beispielhaft Abb. 2a-f;). Der Durchmesser der einzelnen wirkstoffhaltigen Geometrien sollte in jedem Fall kleiner als 6,0 cm sein, bevorzugt 0,2 bis 3,0 cm.
Eine Kombination von Wirkstoffen dergestalt, dass sie nicht gemeinsam in der gleichen Wirkstoffschicht vorliegen, sondern nebeneinander in punktuellen Anordnungen (beispielhaft Abb. 1f) oder in parallelen oder sich kreuzenden Streifen (beispielhaft Abb. 2f) ist vorteilhaft, um durch die sich teilweise sensorisch widersprechenden Reize eine umso stärkere neuronale Antwort und therapeutische Wirkung zu erzielen.
Insgesamt scheint für den Therapieerfolg weniger das genaue Design der wirkstoffhaltigen Geometrien entscheidend zu sein (ähnlich wie die weniger spezifischen Wirkorte bei der Akupunktur), als vielmehr die Tatsache, dass das zu therapierende Feld nicht homogen neuronal-sensorisch stimuliert wird, sondern aufgelockert und in verschiedene Zonen unterschiedlicher Stimuli (oder abwechselnd mit bzw. ohne neuronal-sensorischem Stimulus) aufgeteilt wird. Im Vordergrund steht bei dieser Behandlung letztlich immer das Prinzip der neuronal-sensorischen "Auflockerung", die durch die Geometrie wie auch durch einen Wechsel der therapierten Unterbereiche des Schmerzareals zustande kommt - im Unterschied zu den heute gebräuchlichen flächig abdeckenden, monoton gleichförmigen Stimuli der zur flächigen Abdeckung des Schmerzbereiches gedachten Pflaster.
Als wirkstoffhaltige Klebmasse für die Pflasterstreifen sind sowohl natürliche und synthetische kautschukbasierte, als auch vollsynthetische polymerbasierte Klebmassen denkbar und geeignet. Die Klebmasse muss nicht zwingend vollflächig aufgebracht sein, sondern kann zur Verwirklichung der in den Ansprüchen genannten Geometrien auch rasterpunktförmig aufgebracht werden, beispielsweise durch Siebdruck, wobei die Klebstoffpünktchen auch unterschiedlich groß und/oder unterschiedlich verteilt sein können, oder durch Tiefdruck von in Längs- und Querrichtung zusammenhängenden Stegen. Als Trägermaterialien kommen alle typischerweise im Pflasterbereich eingesetzten Materialien in Frage, sowohl natürlichen als auch synthetischen Ursprungs, egal welcher Verarbeitungstechnologie.
4. Die Zeichnungen zeigen Ausführungsbeispiele.
Die Flächen in Zeichnungen 1 a) und 1 b), jeweils i-iii weisen eine Breite von 0,5 cm bis 6 cm auf bei einer Länge, die mindestens die zweifache Breite beträgt.
Der Abstand zwischen den Streifen in Zeichnung 1c) beträgt mindestens eine Streifenbreite.

In der Zeichnung 1 d) enthalten die schraffiert dargestellten Streifen den Wirkstoff. Es können zwischen 2 und 100 Streifen nebeneinander angebracht sein. Das Ausführungsbeispiel in der Zeichnung zeigt drei Streifen. Die weißen Flächen sind wirkstofffrei. Die Breite der wirkstoffhaltigen Streifen beträgt 0,2 bis 6,0 cm, die Breite der wirkstofffreien Streifen ebenfalls zwischen 0,2 und 6,0 cm.

In der Zeichnung 1 e) entsprechen die Maße den Angaben zu Zeichnung 1 d) mit dem einzigen Unterschied, dass bei den wirkstoffhaltigen Streifen sich jeweils 2 Wirkstoffe abwechseln. In der Zeichnung ist dies dargestellt durch eine unterschiedliche Ausrichtung der Schraffierung, wobei die jeweils identische Schraffierung den identischen Wirkstoff repräsentiert.

In der Zeichnung 1 f) werden zwei unterschiedliche Wirkstoffe A und B jeweils abwechselnd aufgebracht bei einer Anzahl der Wirkstoffstreifen zwischen 2 und 100.

Die Ausführungsbeispiele in Zeichnung 2 a) zeigen unterschiedliche Ausbringungsformen für den Wirkstoff und weisen jeweils eine Breite und Höhe von jeweils 0,2 bis 6,0 cm auf.

Die Zeichnungen 2 b) bis 2 e) zeigen beispielhafte Anordnungen von wirkstoffhaltigen und wirkstofffreien Flächen. Die wirkstoffhaltigen Flächen sind schraffiert dargestellt und weisen eine Größe auf von 0,2 bis 3,0 cm bei einer Gesamtzahl an wirkstoffhaltigen Flächen zwischen 2 und 1000.

Die Zeichnung 2 f) zeigt eine beispielhafte Anordnung von unterschiedlich wirkstoffhaltigen Flächen. Die wirkstoffhaltigen Flächen sind schraffiert dargestellt und weisen eine Größe auf von 0,2 bis 3,0 cm bei einer Gesamtzahl an wirkstoffhaltigen Flächen zwischen 2 und 1000. In diesem Ausführungsbeispiel werden zwei unterschiedliche Wirkstoffe aufgebracht, dargestellt durch unterschiedliche Schraffierungen. Zwischen diesen Flächen sind wirkstofffreie Flächen, hier dargestellt als weiße Bereiche.

Die Zeichnungen 3 a) bis 3 c) zeigen undulierte schwarz dargestellte Ausbringungsformen des Wirkstoffs. Die Streifenbreite beträgt jeweils 0,5 bis 6,0 cm. Die Zeichnungen 3 d) bis 3 f) zeigen unterschiedliche Ausführungsformen, wobei die Gesamtgröße der geometrischen Formen beliebig ist, die Streifenbreite jedoch bevorzugt 0,2 bis 6,0 cm beträgt.

Die Zeichnungen 4a) bis 4 c) zeigen Geometrien mit einer Streifenbreite der schwarz dargestellten Wirkstoffstreifen mit einer Streifenbreite von bevorzugt 0,2 bis 3,0 cm.

Die Zeichnungen 5 a) bis 5 c) zeigen Anwendungsformen, bei denen der Wirkstoff auf dem Gesamtpflaster bzw. der Gesamtfläche ausgebracht wird, diese Fläche jedoch 2 bis 100 Löcher aufweist von einer Größe je Loch zwischen 0,5 und 3,0 cm und die Breite der Brücke sowie zwischen den Löchern jeweils von 0,5 bis 3,0 cm beträgt. In den Zeichnungen sind die wirkstofffreien bzw. gelochten Flächen weiß dargestellt, die wirkstoffhaltigen Bereiche schraffiert.

### Anwendungsbereiche

Anwendung finden kann die Erfindung insbesondere, aber nicht ausschließlich, in folgenden Bereichen:
● Behandlung von Verspannungen oder Schmerz im Schulter- und Nackenbereich
● Behandlung von Verspannungen oder dadurch ausgelöstem Schmerz sonstiger Art
● Behandlung von Schmerzen der unteren Lendenwirbelsäule
● Behandlung von Schmerzen, verursacht durch rheumatische Erkrankungen
● Behandlung von Schmerzen, verursacht durch andere Knochen- und Gelenkschäden (z.B. Arthrosen)
● Behandlung von Schmerzen, ausgelöst durch Prellungen, Zerrungen und besondere muskulärer Beanspruchung ("Muskelkater")
● Behandlung von Schmerzen, verursacht durch Migräne sowie zu deren Vorbeugung
● Behandlung anderer Zustände, für die die Therapie mit den traditionellen Chinesischen Methoden der Akupunktur, Akupressur und Moxibustion anerkannt ist

## Patentansprüche

1. Pflaster mit Wirkstoffbeschichtung (wirkstoffhaltige Klebmasse), bestehend aus einer der folgenden Substanzgruppen, oder aber bestehend aus einer Kombination von
a. lokal die Durchblutung fördernde, das Gefühl von Wärme vermittelnde, Arthrose lindernde, krampflösende oder/und antirheumatische Substanzen, insb. Capsaicin, andere Capsaicioide oder Nonivamid, die u.a. am TRPV1 Kanal wirken, oder Durchblutung fördernde, Wärme vermittelnde ätherische Öle und pflanzliche Extrakte, insb. Ackerminze, Angelika, Angelikawurzel, Anis, Arnika, Baldrian, Basilikum, Bay, Beifuss, Benzoe, Bergamotte, Bohnenkraut, Cajeput, Cananga, Cassia, Davana, Douglasie, Edeltanne, Estragon, Eukalyptus, Fenchel, Fichte, Fichtennadel, Galbanum, Geranie, Gingergrass, Guajakholz, Ingwer, Johanniskraut, Kamille, Kampfer, Kardamom, Kiefer, Kiefernadelöl, Koriander, Kümmel, Latschenkiefer, Lavendel, Lemongrass, Liebstöckel, Limette, Lorbeer, Mairose, Majoran, Mandarine, Manuka, Meerkiefer, Melisse, Minze, Muskatellersalbei, Muskatnuss, Myrte, Nelke, Niaouli, Orange, Oregano, Origanum, Pampelmuse, Pfeffer, Pfefferminze, Rainfarn, Rosenholz, Rosmarin, Römische Kamille, Salbei, Sandelholz, Sassafras, Schafgarbe, Schopflavendel, Sellerie, Tabak, Teebaum, Terpentin, Thuja, Thymian, Wacholder, Wacholderbeere, Weisstanne, Wermut, Wiesenkönigin, Wintergreen, Ysop, Zeder, Zirbelkiefer, Zitrone, Zitronenmelisse, Zypresse
b. lokal das Gefühl von Kälte vermittelnde oder kühlende Substanzen, insb. Menthol, Minzöle, Campher, Methyl Salicylat (Wintergrünöl), andere ätherische Öle und pflanzliche Extrakte, die u.a. am Kälte-Menthol Rezeptor (TRPM8) wirken oder auf sonstige Weise das Gefühl von Kälte vermitteln
c. lokal wirkende, nicht opioide oder auch opioide Analgetika, z.B. Diclofenac oder Lidocain, andere transkutan verwendbare Analgetika zur Behandlung von Schmerzen, insb. Schmerzen bedingt durch Verspannungen aller Art, Rheuma, Prellungen, Zerrungen, Arthrose oder Migräne, **dadurch gekennzeichnet, dass** das Pflaster in einem oder mehr als einem Streifen quer oder längs zur Schmerzrichtung bzw. multilokal punktuell angebracht wird.

2. Pflaster gem. Anspruch 1, **dadurch gekennzeichnet dass** das Verhältnis der längeren zur kürzeren Seite mindestens ein Längenverhältnis von 2:1 aufweist (die längere Seite mindestens zwei Mal so lang ist wie die kürzere; s. beispielhaft Abb. 1 a und 1 b, jeweils Varianten i. bis iii.).

3. Pflaster gem. Anspruch 1 und 2, **dadurch gekennzeichnet, dass** die Pflasterstreifen und/oder die wirkstoffhaltige Klebmasse-Streifen bevorzugt eine Breite von 0,5 bis 6 cm aufweisen (Abb. 1a, 1 b) und das eine beliebige Länge aufweist, von der die jeweils gewünschte Streifenlänge bedarfsgerecht abgeschnitten werden kann; das Pflaster kann hierbei optional Perforationen beliebiger Abstände aufweisen, die das Abtrennen eines Abschnittes der gewünschten Länge ohne Schneidwerkzeug erleichtern.

4. Pflaster gem. Anspruch 1, 2 oder 3, **dadurch gekennzeichnet, dass** das Seitenverhältnis der wirkstoffhaltigen Klebmasse und / oder des Pflasters selbst ein Verhältnis von 0,5 bis 6 cm Breite, bevorzugt 4 cm bis 6 cm, zu einer Länge von 1 cm bis 100 cm, bevorzugt eine Länge zwischen 8 cm und 100 cm aufweist, wobei das Verhältnis der längeren zur kürzeren Seite bevorzugt mindestens einem Verhältnis von 2:1 entspricht.

5. Pflaster gemäß Anspruch 1,2,3 oder 4, **dadurch gekennzeichnet, dass** die Breite der Pflasterstreifen und / oder der Wirkstoff haltigen Klebmasse-Streifen 4,5 bis 5,5 cm beträgt und die Länge 9 bis 100 cm wobei das Verhältnis der längeren zur kürzeren Seite bevorzugt mindestens einem Verhältnis von 2:1 entspricht.

6. Pflaster gemäß Anspruch 1, 2 oder 3, **dadurch gekennzeichnet, dass** die Breite der Pflasterstreifen und / oder der Wirkstoff haltigen Klebmasse-Streifen 0,5 bis 2,5 cm, bevorzugt 1 cm bis 2 cm, beträgt und die Länge 1 bis 100 cm, bevorzugt 2 cm bis 100 cm, wobei das Verhältnis der längeren zur kürzeren Seite bevorzugt mindestens einem Verhältnis von 2:1 entspricht.

7. Pflaster gemäß Anspruch 1, 2 oder 3, **dadurch gekennzeichnet, dass** die Breite der Pflasterstreifen und/oder der Wirkstoff haltigen Klebmasse-Streifen 2,5 bis 4,5 cm, bevorzugt 3 cm bis 4 cm, beträgt und die Länge 5 bis 100 cm, bevorzugt 6 cm bis 100 cm, wobei das Verhältnis der längeren zur kürzeren Seite bevorzugt mindestens einem Verhältnis von 2:1 entspricht.

8. Pflaster gemäß Anspruch 1, 2 oder 3 mit mehreren (2-100) Streifen wirkstoffhaltiger Klebmasse, **dadurch gekennzeichnet, dass** der Abstand zwischen den Streifen bevorzugt eine Breite von 0,2 bis 6 cm aufweist (Abb. 1d-f).

9. Pflaster gemäß Anspruch 1 oder 2, **dadurch gekennzeichnet, dass** das Pflaster quer und längs verlaufende Streifen wirkstoffhaltiger Klebmasse aufweist, die sich orthogonal oder in anderem Winkel kreuzen, wobei die wirkstoffhaltige Klebmasse jedes Streifens eine Breite von 0,2 cm bis 3 cm aufweist und der Abstand zwischen den jeweils parallel verlaufenden Streifen wirkstoffhaltiger Klebmasse ebenso eine Breite von 0,2 cm bis 3 cm aufweist (beispielhaft Abb. 4a-c).

10. Pflaster gemäß Anspruch 1, **dadurch gekennzeichnet, dass** in ein größerflächiges Pflaster mit wirkstoffhaltiger Klebmasse Löcher beliebiger Geometrie (quadratisch, rund etc.) gestanzt werden, wobei die verbleibenden zusammenhängenden Materialbrücken geeignet sind, wahlweise Eigenschaften aus den Ansprüchen zu 3 bis 13 zu erfüllen (Abb. 5a-c).

11. Pflaster gemäß Anspruch 1, **dadurch gekennzeichnet, dass** das Pflaster in einer beliebigen Geometrischen Form eine Länge oder Breite von 6 cm nicht überschreitet (beispielhaft Varianten in Abb. 2a)

12. Pflaster gemäß Anspruch 1, **dadurch gekennzeichnet, dass** das Pflaster eine Mehrzahl (typischerweise 2 bis 1000) punktueller (geometrisch rund/kreisförmig, eckig, auch andere geometrische Formen), räumlich separater Bereiche bzw. Zonen wirkstoffhaltiger Klebmasse aufweist, wobei die Breite jeder Wirkstoffzone zwischen 0,2 bis 3 cm liegt und die Abstände zwischen den Wirkstoffzonen ebenfalls zwischen 0,2 bis 3 cm liegen (Abb. 2b-f).

13. Pflaster gemäß Anspruch 1-3, **dadurch gekennzeichnet dass** die Streifen wirkstoffhaltiger Klebmasse nicht einer Geraden entsprechen, sondern unduliert (wellenförmig), kreisförmig oder anderen Geometrien folgend (Dreiecke, Sterne etc.) verlaufen. Hierbei können sich die durch die Streifen gebildeten geometrischen Figuren teilweise oder vollständig überlappen (z.B. konzentrische Kreise). Die Zahl der geometrischen, nicht geraden Streifen beträgt bevorzugt zwischen 1 und 100; die Breite der Streifen liegt bevorzugt zwischen 0,5 und 6 cm (Abb. 3a-f).

14. Pflaster gemäß Anspruch 1 und Anspruch 9, 10, 12 oder 13, **dadurch gekennzeichnet, dass** die beschriebenen Streifen, Punkte oder andere geometrischen Formen wirkstoffhaltiger Klebmasse abwechselnd verschiedene Substanzen der unter Anspruch 1 genannten Wirkstoffklassen enthalten (Abb. 1f, 2d, 2f, 3c, 3e, 3f).

15. Pflaster gemäß Anspruch 1 und Anspruch 9, 10, 12, 13 oder 14, **dadurch gekennzeichnet, dass** keine Abstände zwischen den wirkstoffhaltigen Streifen, Punkten oder anderen geometrischen Formen bestehen, sondern die jeweiligen Zwischenräume zwischen den Wirkstoffbereichen desselben Wirkprinzips durch Wirkstoffbereiche eines anderen Wirkprinzips ausgefüllt werden (abwechselnde Wirkprinzipien, Abb. 1f).

16. Pflaster gemäß Anspruch 1, **dadurch gekennzeichnet, dass** die wirkstoffhaltige Klebmasse auf eine größer flächige Trägerstruktur in beliebiger Form vermittels Siebdruck oder Tiefdruck aufgebracht wird.

17. Pflaster, gemäß Anspruch 1 bis 16, **dadurch gekennzeichnet, dass** das Pflaster eine von einer neutralen Hautfarbe abweichende, deutlich wahrnehmbare Eigenfarbe aufweist (vorzugsweise Grundfarben, Pastellfarben, Signalfarben, insb. Neon oder Pink).
